(19) Europäisches Patentamt
European Patent Office
Office européen des brevets

(11) **EP 2 306 918 B1**

(12) **EUROPÄISCHE PATENTSCHRIFT**

(45) Veröffentlichungstag und Bekanntmachung des Hinweises auf die Patenterteilung:
**31.08.2016  Patentblatt 2016/35**

(21) Anmeldenummer: **09772062.7**

(22) Anmeldetag: **03.06.2009**

(51) Int Cl.:
***A61B 18/12*** *(2006.01)*

(86) Internationale Anmeldenummer:
**PCT/EP2009/003964**

(87) Internationale Veröffentlichungsnummer:
**WO 2010/000362 (07.01.2010 Gazette 2010/01)**

(54) **ELEKTROCHIRURGISCHES GERÄT ZUM BEHANDELN EINES BIOLOGISCHEN GEWEBES**

ELECTROSURGICAL DEVICE FOR THE TREATMENT OF A BIOLOGICAL TISSUE

DISPOSITIF ÉLECTRO-CHIRURGICAL POUR TRAITER UN TISSU BIOLOGIQUE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **30.06.2008  DE 102008030876
19.08.2008  DE 102008038314**

(43) Veröffentlichungstag der Anmeldung:
**13.04.2011  Patentblatt 2011/15**

(73) Patentinhaber: **ERBE Elektromedizin GmbH
72072 Tübingen (DE)**

(72) Erfinder:
• **FRITZ, Martin
72070 Tübingen (DE)**
• **SCHALL, Heiko
72622 Nürtingen (DE)**

(74) Vertreter: **Bohnenberger, Johannes
Meissner Bolte Patentanwälte
Rechtsanwälte Partnerschaft mbB
Postfach 86 06 24
81633 München (DE)**

(56) Entgegenhaltungen:
**EP-A- 1 707 144       EP-A1- 1 693 014
WO-A-2005/046496     US-A- 5 423 808**

**Beschreibung**

Die Erfindung betrifft einen Elektrochirurgiegenerator zum Behandeln eines biologischen Gewebes, ein Verfahren zum Regeln einer Ausgangsspannung eines elektrochirurgischen Generators und die Verwendung des besagten Elektrochirurgiegenerators.

Ein bekanntes Problem der Hochfrequenzchirurgie sind die Reproduzierbarkeit und Konstanz der Qualität von Schneide- und/oder Koagulationsvorgängen. Es ist bekannt, dass die Qualität der besagten Vorgänge maßgeblich von der hierzu verwendeten Hochfrequenzspannung bzw. von der Intensität der elektrischen Lichtbogen zwischen aktiver Elektrode und Gewebe abhängt. Jedoch werden diese Größen bei kontaktlosen Koagulationsverfahren unter anderem vom Abstand des verwendeten Instruments zu dem Gewebe beeinflusst. Daher ist es wünschenswert, eine Koagulationsvorrichtung und ein entsprechendes Verfahren bereitzustellen, die einen konstanten Lichtbogen unabhängig von der besagten Distanz bereitstellen.

Ein weiteres Problem besteht darin, das Zündverhalten der elektrochirurgischen Geräte zu verbessern. Dies trifft insbesondere für elektrochirurgische Instrumente zu, die ein Edelgas-Plasma erzeugen. Es ist also wünschenswert, ein in bestimmten Grenzen (z. B. 0 mm bis 30 mm) abstandsunabhängiges sicheres Zünden des Plasmas zu gewährleisten. Des Weiteren wird allgemein ein sicherheitsrelevantes Begrenzen des fließenden Stroms gewünscht.

Aus der EP 0 495 140 B1 ist es bekannt, den Wirkanteil des durch einen HF-Generator (Hochfrequenzgenerator) erzeugten Behandlungsstroms mit einem entsprechenden Wirkstromsensor zu erfassen. Eine Stromsteuerungsvorrichtung verarbeitet diese Größe und begrenzt den maximalen Strompegel. In einem weiteren Ausführungsbeispiel schaltet die Stromsteuerungsvorrichtung den HF-Generator ab, wenn der gemessene Strom den eingestellten maximalen Strompegel erreicht oder übersteigt. Durch das Bestimmen des Wirkanteils des Stroms wird sichergestellt, dass unerwünschte Leckströme nicht fälschlicherweise mit berücksichtigt werden, wodurch ein unerwünschtes Abschalten der Vorrichtung verursacht werden könnte.

Aus der DE 25 04 280 ist ein HF-Generator für ein elektrochirurgisches Instrument bekannt, der über eine Steuereinrichtung verfügt, die die Leistung des HF-Generators regelt. Diese Regelung erfolgt auf der Basis der Erfassung eines durch den HF-Behandlungsstrom erzeugten Lichtbogens. Hierfür verfügt die Steuereinrichtung über entsprechende Sensoren.

Aus der WO 00/12019 ist eine Hochfrequenzeinrichtung zur Erzeugung eines Plasmabogens für die Behandlung biologischen Gewebes bekannt. Auch hier wird die Leistung des Generators durch eine Steuereinrichtung, insbesondere durch eine entsprechend ausgelegte Regelschaltung begrenzt. Die Regelschaltung der WO 00/12019 ist ungenau und sehr aufwändig.

Ferner sind ähnliche elektrochirurgische Geräte aus WO 2005/046496, EP 1707144 und EP 1693014 bekannt.

Ausgehend von diesem Stand der Technik ist es Aufgabe der vorliegenden Erfindung, einen verbesserten Elektrochirurgiegenerator zum Behandeln von biologischem Gewebe bereitzustellen. Insbesondere soll ein Elektrochirurgiegenerator bereitgestellt werden, der ein verbessertes Zündverhalten und eine bessere Reproduzierbarkeit der Behandlungsergebnisse aufweist. Des Weiteren sollen ein entsprechendes Verfahren und eine entsprechende Verwendung angegeben werden.

Die Aufgabe wird durch ein elektrochirurgisches Gerät gemäß unabhängigem Anspruch 1 gelöst.

Die vorliegende Erfindung sieht also vor, den tatsächlichen Wirkanteil des HF-Behandlungsstroms zu berechnen und anhand dessen eine Einstellung des Spannungsstellsignals vorzunehmen, so dass jeder beliebige Sollwert einstellbar und einhaltbar ist. Somit eröffnet sich eine Vielzahl von Anwendungsmöglichkeiten. Zum einen können vorgegebene Koalgulations- oder Schneidemodi, die möglicherweise eine bestimmte Frequenz sowie eine bestimmte Leistung voraussetzen, genau eingehalten werden. Des Weiteren kann der Zündvorgang bei kontaktlosen Koagulationsverfahren wesentlich sicherer gestaltet werden. Die Zündung sowie die mittels des entsprechenden Instruments durchgeführte Behandlung sind unabhängig von der Distanz, die zwischen Gewebe und der Elektrode des Instruments vorliegt.

Der Elektrochirurgiegenerator kann eine Leistungsbegrenzungseinrichtung umfassen, welche eine Wirkleistung basierend auf dem Strom- und dem Spannungssignal feststellt und diese auf einen voreingestellten Wert begrenzt. Es erfolgt also eine Leistungsbegrenzung abhängig von der Wirkleistung, die entsprechend der Randbedingungen vorliegt. Hierbei werden insbesondere die Distanz zwischen den Elektroden und/oder zwischen der Elektrode und Gewebe, das verwendete Gerät, die Elektrodengröße usw. berücksichtigt.

Der Elektrochirurgiegenerator kann eine Strombegrenzungseinrichtung umfassen, welche den Wirkanteil des Behandlungsstroms auf einen voreingestellten Wert begrenzt. Vorzugsweise findet also eine Leistungsbegrenzung anhand der Einstellung des Stroms statt.

Der Elektrochirurgiegenerator kann eine Dateneingabe- und/oder Speichervorrichtung zur Eingabe und Speicherung von Wirkwiderstandsanteilen und/oder Blindwiderstandsanteilen von angeschlossenen chirurgischen Instrumenten und Lasten umfassen, wobei die Dateneingabe- und/oder Speichervorrichtung mit der Umwandlungseinrichtung verbunden ist, wobei die Umwandlungseinrichtung zur Einbeziehung der Blindwiderstandsanteile in die Bildung des Wirkanteils des HF-Behandlungsstroms ausgebildet ist.

Somit kann bei der Berechnung des Wirkanteils des HF-Behandlungsstroms die Art des elektrochirurgischen Geräts, insbesondere der spezifische Wirkwiderstandsanteil und/oder Blindwiderstandsanteil berücksichtigt werden. Vorteilhaft ist es, wenn eine Kombination der Wirkungswiderstandsanteile und Blindwiderstandsanteile gespeichert wird. Des Weiteren können Kabel und Leitungen, die in Verbindung mit den entsprechenden Geräten verwendet werden, und Kabellängen berücksichtigt werden. Physikalische Größen, die in einer derartigen Dateneingabe- und/oder Speichervorrichtung gespeichert werden können, sind: Der Widerstand (R), die Induktivität (L) und die Kapazität (C).

Die Umwandlungseinrichtung kann eine Recheneinrichtung umfassen, die das Wirkstromsignal unter Zuhilfenahme einer der folgenden Alternativen bildet:

- eines Hilbert-Transformators;

- einer diskreten Fourier-Transformation (DFT);

- einer schnellen Fourier-Transformation (FFT);

- einer Bildung einer mittleren Leistung, über ($N$) Abtastwerte:

$$P = \frac{1}{N} \sum_{k=1}^{N} u(k) \cdot i(k)$$

sowie einer Bildung von Effektivwerten von Spannung und Strom:

$$u_{eff} = \frac{1}{N} \sqrt{\sum_{k=1}^{N} (u(k))^2}$$

$$i_{eff} = \frac{1}{N} \sqrt{\sum_{k=1}^{N} (i(k))^2}$$

und eines Leistungsfaktors:

$$\cos\varphi = \frac{P}{u_{eff} \cdot i_{eff}}$$

so dass sich das Wirkstromsignal ergibt zu:

$$i_{wirk,eff} = i_{eff} \cdot \cos\varphi$$

Die Recheneinrichtung kann also das Wirkstromsignal vorteilhaft berechnen. Moderne digitale Signalprozessoren sind derart ausgelegt, dass eine Berechnung der entsprechenden Werte, insbesondere des Wirkstromsignals in Echtzeit geschehen kann. Somit können auch aufwändige Signalverarbeitungsoperationen durchgeführt werden, ohne dass es bei der Regelung des HF-Behand-lungsstroms zu einer Verzögerung kommt. Sowohl die Hilber-Transformation, als auch die Fast-Fourier-Transformation und die besagte Bildung einer mittleren Leistung sind geeignet, den Wirkanteil des HF-Behandlungsstroms effektiv und fehlertolerant zu berechnen. Besonders bei einer Verwendung des Verfahrens zur Berechnung des Wirkstroms über die Effektivwerte kann eine rechnerresourcenschonende Implementierung gewährleistet werden. Das Verfahren benötigt verhältnismäßig wenige Operationen um den Wirkanteil zu bestimmen. So kann eine entsprechende Regelschleife häufiger durchlaufen werden, wodurch eine präzisere, schnellere Regelung des Behandlungsstroms erzielt werden kann.

Beispielhaft ist ein Verfahren zum Regeln einer Ausgangsspannung eines elektrochirurgischen Generators offenbart, der einen HF-Behandlungsstrom mit einer HF-Spannung zur Behandlung eines biologischen Gewebes liefert. Das Verfahren umfasst die folgenden Schritte:

- Erfassen des HF-Behandlungsstroms und der HF-Spannung und Erzeugen eines entsprechenden Stromsignales und eines entsprechenden Spannungssignales;

- Bilden eines Wirkanteils des HF-Behandlungsstroms aus dem Stromsignal und dem Spannungssignal und Erzeugen eines entsprechenden Wirkstromsignals;

- Vergleichen des Wirkstromsignals mit einem voreinstellbaren Sollwert und Erzeugen eines Spannungsstellsignals auf Grundlage des Vergleiches;

- Regeln der Ausgangsspannung derart, dass der Wirkanteil des HF-Behandlungsstroms dem Sollwert entspricht.

Das Verfahren hat ähnliche Vorteile wie die erfindungsgemäße Vorrichtung.

Das Verfahren kann das Wirkstromsignal unter Zuhilfenahme einer der nachfolgenden Alternativen berechnen:

- einer Hilbert-Transformation;

- einer diskreten Fourier-Transformation (DFT);

- einer schnellen Fourier-Transformation (FFT);

- einer Bildung einer mittleren Leistung über die Bestimmung von Effektivwerten (siehe oben).

Das Verfahren eignet sich besonders dazu, den Behandlungsstrom für Anwendungen mit Plasmagas zu berechnen.

Des Weiteren wird die Verwendung des besagten elektrochirurgischen Generators offenbart.

Weitere vorteilhafte Ausführungsformen ergeben sich anhand der Unteransprüche.

Nachfolgend wird die Erfindung anhand von einigen Ausführungsbeispielen beschrieben, die mittels Abbildungen näher erläutert werden. Hierbei zeigen:

- Fig. 1     die wesentlichen Komponenten eines erfindungsgemäßen elektrochirurgischen Geräts;

- Fig. 2     eine schematische Darstellung eines Regelkreises des elektrochirurgischen Geräts;

- Fig. 3     ein erstes Verfahren (Hilbertoperation) zur Ermittlung der Wirkleistung und der Blindleistung aus einem Stromsignal und einem Spannungssignal;

- Fig. 4     ein zweites Verfahren (Hilbertoperation) zur Ermittlung der Wirkleistung und der Blindleistung aus einem Stromsignal und einem Spannungssignal;

- Fig. 5     ein drittes Verfahren (FFT) zur Ermittlung der Wirkleistung und der Blindleistung aus einem Stromsignal und einem Spannungssignal.

In der nachfolgenden Beschreibung werden für gleiche und gleichwirkende Teile dieselben Bezugsziffern verwendet.

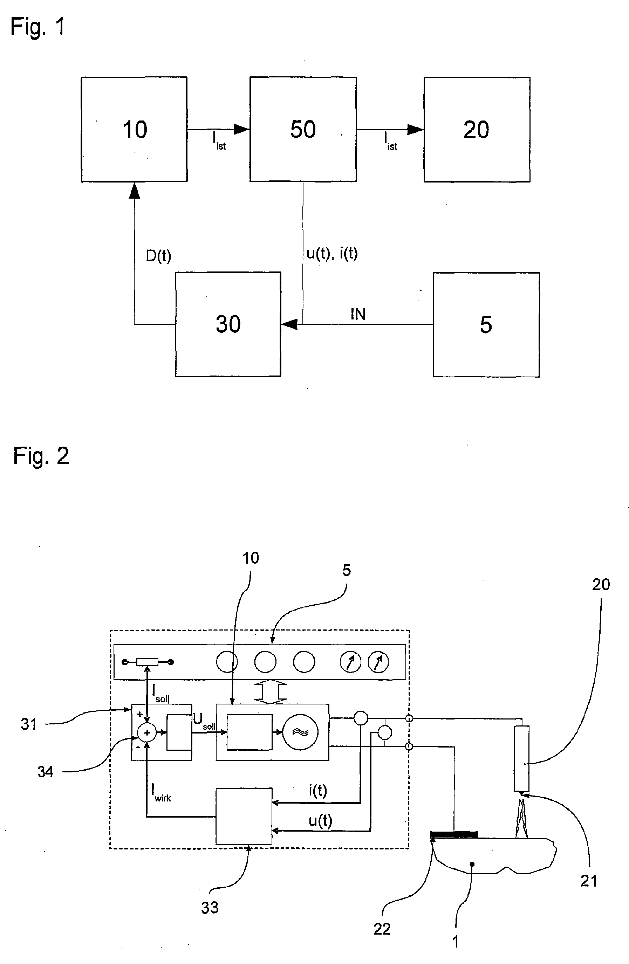

Fig. 1 zeigt die wesentlichen Komponenten eines elektrochirurgischen Instruments. Diese umfassen ein Bedienteil 5 zum Aktivieren und Auswählen eines Schneid- und/oder Koagulationsmodus, ein elektrochirurgisches Instrument 20 zum Applizieren eines HF-Behandlungsstroms $I_{ist}$, einen HF-Generator 10 zum Generieren des HF-Behandlungsstroms $I_{ist}$, eine Messeinrichtung 50 zum Erzeugen eines Stromsignals i(t) und eines Spannungssignals u(t) aus dem erzeugten HF-Behandlungsstrom $I_{ist}$ und eine Steuereinrichtung 30 zur Steuerung des HF-Generators 10.

Allgemein betrachtet stellt der HF-Generator 10 zu einem Zeitpunkt t einen Behandlungsstrom $I_{ist}$ mit einer Ist-Spannung $U_{ist}$ bereit, mit der das elektrochirurgische Instrument 20 betrieben wird. Aus diesem Wert ermittelt die Messeinrichtung 50 die für diese Scheinleistung S charakteristischen Stromsignale i(t) und Spannungssignale u(t). Die Steuereinrichtung 30 verarbeitet das Stromsignal i(t) und das Spannungssignal u(t) sowie die Bedienersignale IN, die der Benutzer des elektrochirurgischen Instruments 20 mit dem Bedienteil 5 eingibt. Anhand dieser Signale ermittelt die Steuereinrichtung 30 entsprechende Steuersignale D(t), auf Grund derer der HF-Generators 10 eingestellt wird. Diese Steuersignale D (t) umfassen ein Spannungsstellsignal $U_{soll}$. Um eine angemessene Steuerung des HF-Generators 10 zu ermöglichen, umfasst die Steuereinrichtung 30 einen Prozessor, der dazu ausgelegt ist, verschiedene Operationen zur Signalverarbeitung des Stromsignals i(t) und des Spannungssignals u(t) durchzuführen, sowie eine entsprechende Speichereinrichtung, die es ermöglicht, Ergebnisse und/oder Einstellungen und/oder weitere Daten kurzfristig oder dauerhaft zu speichern. Die Steuereinrichtung 30 ist also unter anderem dazu ausgelegt, den weiter unten beschriebenen Regler 31 und einen entsprechenden Regelkreislauf zu implementieren.

Fig. 2 zeigt schematisch einen Regelkreislauf, der bei einer erfindungsgemäßen Steuerung des HF-Generators 10 angewandt wird. Der HF-Generator 10 betreibt ein monopolares elektrochirurgisches Instrument 20, das über eine erste Leitung mit diesem verbunden ist. Das elektrochirurgische Instrument 20 umfasst eine erste Elektrode 21 zur Applikation eines HF-Behandlungsstroms $I_{ist}$. Die zweite Elektrode 22, die ebenfalls über eine Leitung mit dem HF-Generator 10 verbunden ist, liegt unmittelbar an dem zu behandelnden Gewebe 1 an. Es handelt sich um eine Neutralelektrode, die einen großflächigen Kontakt mit dem zu behandelnden Gewebe 1 bzw. dem Körper des Patienten herstellt.

In einem Durchlauf des Regelkreislaufs wird durch das Bedienteil 5 ein Sollwert des Wirkstroms $I_{soll}$ vorgegeben. Ein Regler 31 ermittelt mittels eines Komparators oder eines Fehlerverstärkers 34 aus diesem Sollwert des Wirkstroms $I_{soll}$ und einem Wirkstrom $I_{wirk}$ ein Spannungsstellsignal $U_{soll}$. Der HF-Generator 10 legt eine entsprechende Spannung an die Elektroden 21, 22 an. Es ergibt sich ein Behandlungsstrom $I_{ist}$. Die Messeinrichtung 50 greift das Stromsignal i(t) und das Spannungssignal u(t) ab und ermittelt mittels einer Wirkstromberechnungseinheit 33 einen Wirkstrom $I_{wirk}$. Der Wirkstrom $I_{wirk}$ wird, wie bereits erwähnt, in dem besagten Fehlerverstärker 34 mit dem eingestellten Sollwert des Behandlungsstrom $I_{soll}$ verglichen und in ein entsprechendes Spannungsstellsignal $U_{soll}$ umgewandelt. Dieses Spannungsstellsignal $U_{soll}$ wird, wie bereits beschrieben, dem HF-Generator 10 zugeführt. Es ergibt sich also eine Regelschleife, die fortlaufend aus dem HF-Behandlungsstrom $I_{ist}$ den Wirkstrom $I_{wirk}$ bestimmt und das Spannungsstellsignal $U_{soll}$ derart einstellt, dass der Differenzbetrag zwischen dem Wirkstrom $I_{wirk}$ und dem Sollwert des Wirkstroms $I_{soll}$ möglichst gering ist.

Ein wesentlicher Teil der vorliegenden Erfindung besteht darin, den Wirkstrom $I_{wirk}$ oder etwas allgemeiner formuliert das Verhältnis zwischen Wirkleistung P und Blindleistung Q des vorher beschriebenen Systems genau und fehlertolerant zu bestimmen. Hierfür werden im Folgenden vier unterschiedliche Verfahren angegeben.

Die Berechnung des Wirkstroms $I_{wirk}$ führt zunächst über die Bildung eines Leisungsfaktors cos (p, der den Wirkstrom $I_{wirk}$ und den Scheinstrom $I_{schein}$ in eine Relation setzt. Im vorab beschriebenen Ausführungsbeispiel entspricht der Scheinstrom $I_{schein}$ dem Behandlungsstrom $I_{ist}$. Es ergibt sich:

$$I_{wirk} = I_{schein} \cos \cdot \varphi \qquad \text{(Formel 1)}$$

Gemäß dem Leistungsdreieck ergibt sich folgender Zusammenhang zwischen Wirkleistung P, Blindleistung Q und Scheinleistung S:

$$S = \sqrt{P^2 + Q^2} \qquad \text{(Formel 2)}$$

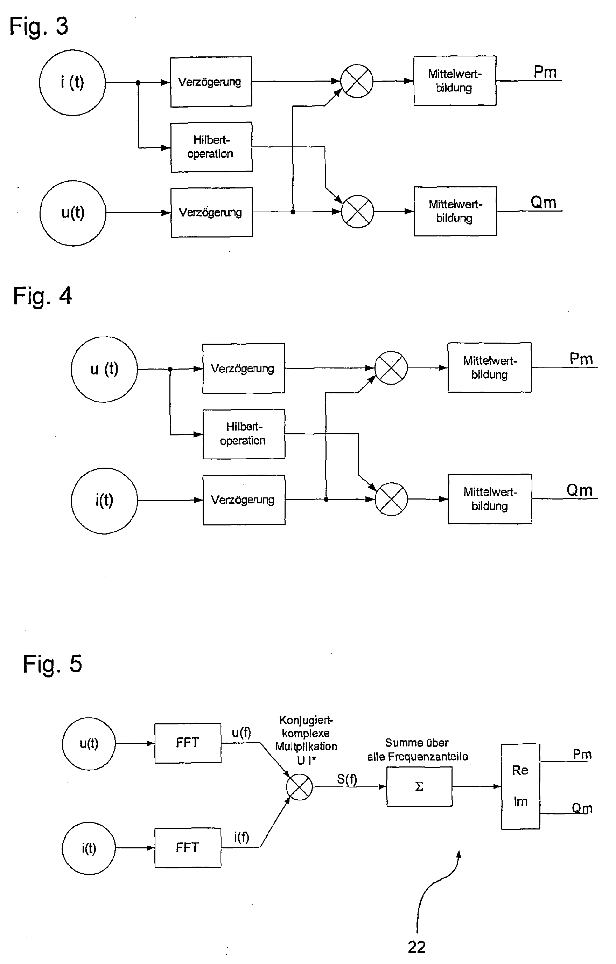

In einem ersten Ausführungsbeispiel wird zur Berechnung der Wirkleistung P und der Blindleistung Q die Hilbert-Transformation verwendet (vergleiche Fig. 3). Der bekannte Hilbert-Transformator bewirkt eine frequenzunabhängige und amplitudenneutrale Phasenverschiebung von Zeitsignalen um 90°. Bei der Weiterverarbeitung des Signals ist allerdings darauf zu achten, dass der digital als FIR-Filter realisierte Hilbert-Operator eine für FIR-Strukturen typische Durchlaufzeit aufweist. Es muss daher darauf geachtet werden, dass stets zeitlich zugehörige Werte von Strom und Spannung miteinander weiter verarbeitet werden. In dem ersten Ausführungsbeispiel erfolgt eine Ermittlung eines Blindleistungsmittelwertes $Q_m$ durch die Anwendung des Hilbert-Operators auf das zeitabhängige Stromsignal i(t) und anschließender Multiplikation mit dem zeitverzögerten Spannungssignal u(t). Durch Mittelwertsbildung über mindestens eine Spannungs- oder Stromverlaufsperiode ist der Mittelwert der Blindleistung Q, also der Blindleistungsmittelwert $Q_m$ erhältlich. Die Berechnung des Wirkleistungsmittelwerts $P_m$ erfolgt durch direkte Multiplikation des Stromsignals i(t) und des Spannungssignals u(t), wobei auch hier über mindestens eine Periode gemittelt wird.

Durch Auswertung der Beziehungen im rechtwinkligen Leistungsdreieck (Scheinleistung S ist Hypotenuse, Wirkleistung P ist Ankathete und Q ist Gegenkathete zum Winkel φ) kann man den Leistungsfaktor cos φ auf die folgenden zwei Arten berechnen (vergleiche auch Formel 1):

$$\cos \varphi = \cos\left(\arctan\left(\frac{Q_m}{P_m}\right)\right) \qquad \text{(Formel 3)}$$

$$\cos\varphi = \frac{P_m}{\sqrt{P_m^2 + Q_m^2}} \qquad \text{(Formel 4)}$$

Die beschriebene Steuerung 30 kann also unter Verwendung der Hilbert-Transformation und mittels der Formeln 1 und 3 oder 1 und 4 den Wirkstrom $I_{wirk}$ berechnen.

Ein zweites Verfahren zur Berechnung der Wirkleistung P und der Blindleistung Q kann ebenfalls mittels der Hilbert-Transformation (vergleiche Fig. 4) bereitgestellt werden. Hier wird das Spannungssignal u(t) transformiert und mit dem verzögerten Stromsignal i(t) multipliziert. Der Blindleistungsmittelwert $Q_m$ ergibt sich durch eine Mittlung der so berechneten Werte. Der Wirkleistungsmittelwert $P_m$ ergibt sich durch eine Multiplikation des Stromsignals i(t) und des Spannungssignals u(t) und einer anschließenden Mittlung der Werte.

Anhand des Blindleistungsmittelwerts $Q_m$ und des Wirkleistungsmittelwerts $P_m$ lässt sich mit der oben aufgezeigten Formel der Leistungsfaktor cos $\varphi$ ermitteln, wodurch eine Beziehung zwischen dem Wirkstrom $I_{wirk}$ und dem Scheinstrom $I_{schein}$ hergestellt werden kann.

Ein drittes Verfahren zur Ermittlung der Wirkleistung P ergibt sich gemäß Fig. 5. Zunächst wird das Stromsignal i(t) und das Spannungssignal u(t) einer diskreten oder schnellen Fourier-Transformation (DFT, FFT) unterzogen. Um den Rechenaufwand zu minimieren, wird vorzugsweise eine FFT gewählt. Als Ergebnis der FFT erhält man einen Vektor von komplexen Zahlenwerten, bestehend aus Real- und Imaginäranteilen des Stromsignals i(t).

Nach einer konjugiert komplexen Multiplikation ergibt sich die Leistung, getrennt in Wirk- (P) und Blindanteil (Q):

$$\underline{U} \cdot \underline{I}^* = |U|e^{j\varphi_u} \cdot |I|e^{-j\varphi_i} = |U| \cdot |I|e^{-j(\varphi_u - \varphi_i)} = P + jQ \qquad \text{(Formel 5)}$$

Durch ein Aufsummieren der Vektorwerte der Wirkleistung P und ein getrenntes Aufsummieren der Blindleistung Q erhält man den Wirkleistungsmittelwert $P_m$ und den Blindleistungsmittelwert $Q_m$. Der Leistungsfaktor cos $\varphi$ kann wie bereits weiter oben beschrieben unter Verwendung der Formeln 3 oder 4 ermittelt werden.

Erfindungsgemäß ergeben sich zwei Verfahren zur Ermittlung des Scheinstroms $I_{schein}$. Wert des Scheinstroms $I_{schein}$ kann aus den komplexwertigen Ergebnissen der FFT durch Betragsbildung ermittelt werden:

$$I_{schein} = \sum_{alle: Frequenzanteile} |i_r + j \cdot i_i| = \sum_{alle: Frequenzanteile} \sqrt{i_r^2 + i_i^2} \qquad \text{(Formel 6)}$$

Alternativ kann eine Aufsummierung der reelwertigen Augenblickswerte des Stroms über eine Periode vorgenommen werden.

In einem vierten Verfahren kann der Wirkstrom P aus den Effektivwerten von Strom $i_{eff}$ und Spannung $u_{eff}$ ermittelt werden. Für sinusförmige Strom- und Spannungsverläufe ist die Wirkleistung P über den Leistungsfaktor cos $\varphi$ wie folgt definiert:

$$P = u_{eff} \cdot i_{eff} \cdot \cos\varphi \qquad \text{(Formel 7)}$$

Ersetzt man die Wirkleistung P durch den Wirkleistungsmittelwert $P_m$, so lässt sich der Leistungsfaktor cos $\varphi$ aus dem Wirkleistungsmittelwert $P_m$ und den Effektivwerten der Strom- und Spannungszeitverläufe i(t), u(t) ermitteln. Für den Leistungsfaktor cos $\varphi$ ergibt sich dann:

$$\cos\varphi = \frac{P_m}{u_{eff} \cdot i_{eff}} \qquad \text{(Formel 8)}$$

Eine Berechnung des Wirkleistungmittelwerts $P_m$ lässt sich bei N Abtastwert wie folgt ermitteln:

$$P = \frac{1}{N} \sum_{k=1}^{N} u(k) \cdot i(k) \qquad \text{(Formel 9)}$$

Der Effektivwert $u_{eff}$ der Spannung ergibt sich über N Abtastwerte aus dessen Definitionsgleichung:

$$u_{eff} = \frac{1}{N} \sqrt{\sum_{k=1}^{N} (u(k))^2}$$

(Formel 10)

Auf analoge Weise lässt sich der Effektivwert des Stroms $i_{eff}$ bestimmen:

$$i_{eff} = \frac{1}{N} \sqrt{\sum_{k=1}^{N} (i(k))^2}$$

(Formel 11)

Mit Hilfe des so gewonnenen Leistungsfaktors cos φ ist der Wirkanteil des Stroms:

$$i_{wirk,eff} = i_{eff} \cdot \cos\varphi$$

(Formel 12)

Das letzt genannte Verfahren zeichnet sich besonders dadurch aus, dass hier verhältnismäßig wenige Operationen durchgeführt werden müssen, um den Wirkstrom $I_{wirk}$ zu ermitteln. Jedoch lassen mit den heutigen digitalen Signalverarbeitungsprozessoren auch die Verfahren 1 bis 3 implementieren.

Bezugszeichenliste

| | |
|---|---|
| 1 | Gewebe |
| 5 | Bedienteil |
| 10 | HF-Generator |
| 20 | elektrochirurgisches Instrument |
| 21 | erste Elektrode |
| 22 | zweite Elektrode |
| 30 | Steuereinrichtung |
| 31 | Regler |
| 33 | Wirkstromberechnungseinheit |
| 34 | Fehlerverstärker |
| 50 | Messeinrichtung |
| $S(t)$ | Scheinleistung |
| $D(t)$ | Steuersignal |
| $I_{ist}$ | Behandlungsstrom |
| $I_{soll}$ | Sollwert des Wirkstroms |
| $I_{schein}$ | Scheinstrom |
| $I_{wirk}$ | Wirkstrom |
| $U_{ist}$ | Ist-Spannung |
| $U_{soll}$ | Spannungsstellsignal |
| IN | Bedienersignal |
| S | Scheinleistung |
| Q | Blindleistung |
| $Q_m$ | Blindleistungsmittelwert |
| P | Wirkleistung |
| $P_m$ | Wirkleistungsmittelwert |
| $i(t)$ | Stromsignal |
| $i_{eff}$ | Effektivwert des Stroms |
| $u(t)$ | Spannungssignal |
| $u_{eff}$ | Effektivwert der Spannung |
| cos φ | Leistungsfaktor |

**Patentansprüche**

1. Elektrochirurgisches Gerät zum Behandeln, z.B. Schneiden oder Koagulieren eines biologischen Gewebes mit einem elektrochirurgischen Instrument zum Erzeugen eines Edelgas-Plasma und einem Elektrochirurgiegenerator, wobei der Elektrochirurgiegenerator umfasst:

   - einen Generatorteil (10), der einen HF-Behandlungsstrom ($I_{ist}$) mit einer entsprechend einem Spannungsstell-signal ($U_{soll}$) eingestellten HF-Spannung so liefert, dass dem biologischen Gewebe elektrische Energie mittels des Instruments zuführbar ist;
   - eine Messeinrichtung zum Erfassen des HF-Behandlungsstromes und der HF-Spannung zum Erzeugen eines entsprechenden Stromsignals (i(t)) und eines entsprechenden Spannungssignals (u(t));
   - eine Umwandlungseinrichtung, welcher das Stromsignal (i(t)) und das Spannungssignal (u(t)) zugeführt werden und die derart ausgebildet ist, dass aus dem Stromsignal (i(t)) und dem Spannungssignal (u(t)) ein dem Wirkanteil des HF-Behandlungsstromes ($I_{ist}$) entsprechendes Wirkstromsignal gebildet wird;

   **dadurch gekennzeichnet, dass** das elektrochirurgische Gerät ferner eine Regeleinrichtung aufweist, welche dazu ausgebildet ist, das Wirkstromsignal mit einem voreinstellbaren Sollwert ($I_{soll}$) zu vergleichen und auf Grundlage des Vergleiches das Spannungsstellsignal ($U_{soll}$) derart zu erzeugen, dass der Wirkanteil des HF-Behandlungsstromes entsprechend dem Sollwert einstellbar ist, um ein sicheres und vom Abstand zum biologischen Gewebe unabhängiges Zünden des Edelgas-Plasma zu gewährleisten.

2. Elektrochirurgisches Gerät nach Anspruch 1,
   **dadurch gekennzeichnet, dass**
   eine Leistungsbegrenzungseinrichtung vorgesehen ist, welche eine Wirkleistung basierend auf dem Strom (i(t)) und dem Spannungssignal (u(t)) feststellt und diese auf einen voreingestellten Wert begrenzt.

3. Elektrochirurgisches Gerät nach einem der vorhergehenden Ansprüche,
   **dadurch gekennzeichnet, dass**
   eine Strombegrenzungseinrichtung vorgesehen ist, welche den Wirkanteil des Behandlungsstromes auf einen voreingestellten Wert begrenzt.

4. Elektrochirurgisches Gerät nach einem der vorhergehenden Ansprüche,
   **dadurch gekennzeichnet, dass**
   eine Dateneingabe- und/oder Speichervorrichtung zur Eingabe und Speichern von Wirkwiderstandsanteilen und/oder Blindwiderstandsanteilen von angeschlossenen chirurgischen Instrumenten (20) und Lasten vorgesehen und mit der Umwandlungseinrichtung verbunden ist, wobei die Umwandlungseinrichtung zur Einbeziehung der Blindwiderstandsanteile in die Bildung des Wirkanteils des HF-Behandlungsstromes ($I_{ist}$) ausgebildet ist.

5. Elektrochirurgisches Gerät nach einem der vorhergehenden Ansprüche,
   **dadurch gekennzeichnet, dass**
   die Umwandlungseinrichtung eine Recheneinrichtung umfasst, die das Wirkstromsignal bildet unter Zuhilfenahme einer der folgenden Alternativen:

   - eines Hilbert-Transformators;
   - einer diskreten Fourier-Transformation (DFT);
   - einer schnellen Fourier-Transformation (FFT);
   - einer Bildung einer mittleren Leistung,
   - über (N) Abtastwerte:

$$P = \frac{1}{N}\sum_{k=1}^{N} u(k) \cdot i(k)$$

   sowie einer Bildung von Effektivwerten von Spannung und Strom:

$$u_{eff} = \frac{1}{N} \sqrt{\sum_{k=1}^{N} (u(k))^2}$$

$$i_{eff} = \frac{1}{N} \sqrt{\sum_{k=1}^{N} (i(k))^2}$$

und eines Leistungsfaktors:

$$\cos \varphi = \frac{P}{u_{eff} \cdot i_{eff}}$$

so dass sich das Wirkstromsignal ergibt zu:

$$i_{wirk,eff} = i_{eff} \cdot \cos \varphi$$

**Claims**

1. Electrosurgical device for treating, e.g. cutting or coagulating, a biological tissue, comprising an electrosurgical instrument for generating a noble gas plasma and an electrosurgical generator, wherein the electrosurgical generator comprises:

   - a generator part (10), which supplies an RF treatment current ($I_{actual}$) with an RF voltage adjusted in accordance with a voltage control signal ($U_{setpoint}$) in such a way that electric energy is guidable to the biological tissue by means of the instrument;
   - a measuring apparatus for detecting the RF treatment current and the RF voltage for generating a corresponding current signal (i(t)) and a corresponding voltage signal (u(t));
   - a conversion apparatus, to which the current signal (i(t)) and the voltage signal (u(t)) are supplied and which is embodied in such a way that an active current signal corresponding to the active component of the RF treatment current ($I_{actual}$) is formed from the current signal (i(t)) and the voltage signal (u(t));

   **characterized in that** the electrosurgical device furthermore has a closed-loop control apparatus, which is embodied to compare the active current signal with a pre-adjustable setpoint value ($I_{setpoint}$) and generate the voltage control signal ($U_{setpoint}$) on the basis of the comparison in such a way that the active component of the RF treatment current is adjustable in accordance with the setpoint value in order to ensure safe ignition of the noble gas plasma which is independent of the distance to the biological tissue.

2. Electrosurgical device according to Claim 1,
   **characterized in that**
   a power limiting apparatus is provided, which determines an active power on the basis of the current (i(t)) and the voltage signal (u(t)) and limits these to a preset value.

3. Electrosurgical device according to one of the preceding claims,
   **characterized in that**
   a current limiting apparatus is provided, which limits the active component of the treatment current to a preset value.

4. Electrosurgical device according to one of the preceding claims,
   **characterized in that**
   a data input and/or data storage device is provided for entering and storing equivalent resistance components and/or reactance components of connected surgical instruments (20) and loads, and for connection to the conversion apparatus, the conversion apparatus being embodied to include the reactance components in the formation of the active component of the RF treatment current ($I_{actual}$).

5. Electrosurgical device according to one of the preceding claims,
   **characterized in that**
   the conversion apparatus comprises a computer apparatus, which forms the active current signal with the aid of one of the following alternatives:

   - a Hilbert transformer;
   - a discrete Fourier transform (DFT);
   - a fast Fourier transform (FFT);
   - forming a mean power,
   - over (N) sampled values:

   $$P = \frac{1}{N} \sum_{k=1}^{N} u(k) \cdot i(k)$$

   and forming effective values of voltage and current:

   $$u_{eff} = \frac{1}{N} \sqrt{\sum_{k=1}^{N} (u(k))^2}$$

   $$i_{eff} = \frac{1}{N} \sqrt{\sum_{k=1}^{N} (i(k))^2}$$

   and a power factor:

   $$\cos \varphi = \frac{P}{u_{eff} \cdot i_{eff}}$$

   such that the active current signal emerges as:

   $$i_{active,eff} = i_{eff} \cdot \cos \varphi .$$

**Revendications**

1. Dispositif électro-chirurgical de traitement, par exemple d'incision ou de coagulation d'un tissu biologique avec un instrument électro-chirurgical permettant de générer un plasma de gaz rare et un générateur électro-chirurgical, le générateur électro-chirurgical comprenant :

   - un élément générateur (10), qui fournit un courant de traitement HF ($I_{réel}$) avec une tension HF réglée en fonction d'un signal de réglage de tension ($U_{théo}$) de manière à ce que de l'énergie électrique puisse être acheminée au tissu biologique au moyen de l'instrument ;
   - un dispositif de mesure pour détecter le courant de traitement HF et la tension HF afin de générer un signal de courant correspondant (i(t)) et un signal de tension correspondant (u(t)) ;
   - un dispositif de conversion auquel le signal de courant (i(t)) et le signal de tension (u(t)) sont envoyés et qui est conçu de manière à former à partir du signal de courant (i(t)) et du signal de tension (u(t)) un signal de courant actif correspondant à la partie active du courant de traitement HF ($I_{réel}$);

   **caractérisé en ce que** le dispositif électro-chirurgical présente en outre un dispositif de réglage qui est conçu pour comparer le signal de courant actif à une valeur théorique pré-réglable ($I_{\text{théo}}$) et générer le signal de réglage de tension ($U_{\text{théo}}$) sur la base de la comparaison de manière à ce que la partie active de courant de traitement HF soit réglable en fonction de la valeur théorique afin de garantir un allumage sûr et indépendant de la distance par rapport au tissu biologique du plasma de gaz rare.

2. Dispositif électro-chirurgical selon la revendication 1,
**caractérisé en ce**
**qu'**il est prévu un dispositif de limitation de puissance qui constate une puissance active en se basant sur le courant ($i(t)$) et le signal de tension ($u(t)$) et limite ceux-ci a une valeur pré-paramétrée.

3. Dispositif électro-chirurgical selon une des revendications précédentes,
**caractérisé en ce**
**qu'**il est prévu un dispositif de limitation de courant qui limite la partie active du courant de traitement à une valeur pré-paramétrée.

4. Dispositif électro-chirurgical selon une des revendications précédentes,
**caractérisé en ce**
**qu'**un dispositif de saisie et/ou d'enregistrement de données est prévu pour saisir et sauvegarder des pourcentages de résistance active et/ou des pourcentages de résistance aveugle d'instruments chirurgicaux raccordés (20) et de charges et qu'il est relié au dispositif de conversion, le dispositif de conversion étant conçu pour intégrer des pourcentages de résistance aveugle dans le calcul de la part active du courant de traitement HF ($I_{\text{réel}}$).

5. Dispositif électro-chirurgical selon une des revendications précédentes,
**caractérisé en ce que**
le dispositif de conversion comprend un dispositif de calcul qui calcule le signal de courant actif en s'aidant d'une des alternatives suivantes :

    - un transformateur Hilbert ;
    - une transformation Fourier discrète (DFT) ;
    - une transformation Fourier rapide (FFT) ;
    - un calcul de puissance moyenne
    - via (N) valeurs de balayage :

$$P = \frac{1}{N} \sum_{k=1}^{N} u(k) \cdot i(k)$$

et un calcul de valeurs effectives de tension et de courant :

$$u_{\textit{eff}} = \frac{1}{N} \sqrt{\sum_{k=1}^{N} (u(k))^2}$$

$$i_{\textit{eff}} = \frac{1}{N} \sqrt{\sum_{k=1}^{N} (i(k))^2}$$

et un facteur de puissance :

$$\cos\varphi = \frac{P}{u_{\textit{eff}} \cdot i_{\textit{eff}}}$$

de sorte qu'on obtient le signal de courant actif suivant :

$$i_{wirk,eff} = i_{eff} \cdot \cos \varphi$$

$$i_{wirk,eff} = i_{eff} \cdot \cos \varphi$$

Fig. 1

Fig. 2

Fig. 3

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0495140 B1
- DE 2504280
- WO 0012019 A
- WO 2005046496 A
- EP 1707144 A
- EP 1693014 A